# EUROPEAN PATENT SPECIFICATION

(11) **EP 2 775 838 B1**
(45) Date of publication and mention of the grant of the patent: **27.09.2017**
(21) Application number: 12783249.1
(22) Date of filing: 24.10.2012
(51) Int. Cl.: A01N 37/08, A01N 65/00, A01N 25/04, A01N 37/36, A01N 65/06, A61K 9/00, A61K 47/10, A61K 9/08, A61K 31/192, C08L 93/00, D21H 21/36, A01P 1/00, A61K 36/13, B27K 3/15

(54) **AQUEOUS ANTIMICROBIAL COMPOSITION CONTAINING CONIFEROUS RESIN ACIDS**
WÄSSERIGE ANTIMIKROBIELLE ZUSAMMENSETZUNG ENTHALTEND DIE HARZSEÜREN DER KONIFERE
COMPOSITION ANTIMICROBIENNE AQUEUSE CONTENANT DES ACIDES DE RÉSINES DE CONIFÈRES

(30) Priority: 26.10.2011 FI 20110371; 29.08.2012 FI 20120287
(43) Date of publication of application: 17.09.2014
(73) Proprietor: Patolab Oy, 02160 Espoo (FI)
(72) Inventor: SIPPONEN, Pentti, FI-02160 Espoo (FI)
(74) Representative: Kolster Oy Ab
(86) International application number: PCT/FI2012/051019
(87) International publication number: WO 2013/060936

(56) References cited:
- WO-A2-2011/042613
- CN-A- 101 804 044
- GB-A- 1 419 116
- KR-A- 20060 104 161
- RO-B1- 107 087
- RO-B1- 119 814
- US-A- 2 128 973
- US-A1- 2011 039 816
- DATABASE WPI Week 200702 Thomson Scientific, London, GB; AN 2007-012677 XP002690674, & JP 2006 312590 A (SUNSTAR CHEM IND CO LTD) 16 November 2006 (2006-11-16)
- M RAUTIO ET AL: "Antibacterial effects of home-made resin salve from Norway spruce (Picea abies)", APMIS, vol. 115, 10 May 2007 (2007-05-10), pages 335-340, XP055049882, DOI: 10.1111/j.1600-0463.2007.apm_548.x/pdf
- THOR A SOEDERBERG ET AL: "Antibacterial Activity of Rosin and Resin Acids in Vitro", SCANDINAVIAN JOURNAL OF PLASTIC AND RECONSTRUCTIVE SURGERY, STOCKHOLM, SE, vol. 24, no. 3, 1 January 1990 (1990-01-01), pages 199-205, XP009164502, ISSN: 0036-5556
- J. M. Mcguire ET AL: "Gas Chromatographic Analysis of Tall Oil Fractionation Products After Methylation with N,N-Dimethylformamide Dimethylacetal", JOURNAL OF CHROMATOGRAPHIC SCIENCE, vol. 36, no. 2, 1 February 1998 (1998-02-01), pages 104-108, XP055277161, Cary, NC, USA ISSN: 0021-9665, DOI: 10.1093/chromsci/36.2.104

## Description

### Field of the invention

The present invention relates to an aqueous antimicrobial composition comprising coniferous resin acids, a method for its preparation and its use as an antimicrobial agent in medical and non-medical field.

### Background of the invention

Coniferous resin, typically of pine or spruce origin, has been widely used in naturopathy and traditional medicine for healing of skin wounds and skin infections. In this practice, the resin was typically cooked into animal fat.

Coniferous resin is a mixture of various chemical compounds, mainly composed of resin acids, lignans, fatty acids and volatile terpenic compounds. In addition to the term 'resin', such terms as 'rosin', 'rosin gum' or 'colophonium' are widely used as synonyms for the resin naturally present in coniferous trees.

The antimicrobial characteristics, such as antifungal and antibacterial characteristics, and even antiviral characteristics of coniferous resin are widely described in the literature. It has been shown that particularly the resin acids of the resin have antimicrobial potentials. For example, coniferous resin has broad-spectrum antimicrobial characteristics against pathogenic gram-positive and gram-negative bacteria. Indeed, there are fat-based resin salves on the market that are effective in the treatment of skin wounds and fungal nail infections. These salves are prepared by dissolving resin to ethanol.

WO 2011/042613 A2 describes an antimicrobial composition comprising coniferous resin acids and/or their derivates in alcohol solutions.

KR 2006 0104161 discloses a cosmetic composition comprising pine cone extract having antibiotic and antifungal activity in an amount of 0.01-20.0 wt.%.

US 2128973 A discloses a self-emulsifying composition comprising rosin for coating fruits, nuts, nursery stock and the like for reducing desiccation and decay.

GB 1419116 A discloses shampoos containing 1-25% of rosin.

JP2006312590A discloses a composition for mouth containing 0.001 to 20 weight% of dehydroabietic acid.

RO 119814 B1 discloses cosmetic gels containing 0.1-5% of purified coniferous resin.

(RO 107087 B1 discloses a therapeutic ointment for topical treatment of wounds containing 5-15% pine resin.

M. Rautio el al.; "Antibacterial effects of home-made resin salve from Norway spruce (Picea abies)", APMIS, vol. 115, 2007, pages 335-340, describes antibacterial effects of resin salve from Norway spruce.

CN 101 804 044 discloses a use of a salt of abietic acid for suppressing Helicobacter pylori.

US 2011/039816 A1 discloses a use of an extract from pine and spruce green needles (CGNC) for inhibiting Helicobacter pylori growth.

T.A. Söderberg et al., "Antibacterial Activity of Rosin and Resin Acid in Vitro", Scandinavian Journal of Plastic and Reconstructive Surgery, vol. 24. No. 3, 1990, pages 1195-205, describes antimicrobial activity of Portuguese rosin, tall oil rosin and pure resin acids.

The coniferous resin, and particularly resin acids contained therein, is poorly soluble in water. An extensive use of the coniferous resin as an antimicrobial agent in a wide variety of materials and products is not possible until an aqueous solution of resin can be provided.

### Brief description of the invention

We have now found that a stable, uniformly distributed aqueous solution of coniferous resin acids as defined in claim 1 comprising hydroxylated derivatives of dehydroabietic acid and a dispersing agent can be prepared. We have surprisingly found that such an aqueous solution exhibit antimicrobial characteristic even with only 1 to 100 ppm of coniferous resin acids. The aqueous solution of the resin acids can be beneficially introduced to other
aqueous compositions used in the treatment of a mammal, such as in pharmaceutical formulations, saline solutions, sugar solutions, aqueous infusion and injections solutions, rinsing solutions, clysma, water-dilutable salves, lotions, and cosmetic products, and to other products used in non-medical field, without losing an antimicrobial effect thereof.

An object of the present invention is an aqueous antimicrobial composition comprising coniferous resin acids, a dispersing agent and an aqueous medium, wherein an amount of the coniferous resin acids of the composition is in the range of 1 to 100 ppm, the coniferous resin acids comprise 7α-hydroxydehydroabietic acid, 7α-hydroxydehydroabietic acid, 15-hydroxydehydroabietic acid, 7β,15-dihydroxy-dehydroabietic acid, 7α,15-dihydroxydehydroabietic acid, 18-hydroxydehydroabietic acid, further hydroxylated derivates of dehydroabietic acid or a mixture thereof, and are provided as or derived from a resin of coniferous trees or provided as or derived from a resin acid fraction of tall oil obtained from kraft pulping of coniferous trees, and the dispersing agent is selected from a group consisting of polyhydric alicyclic alcohol, polyhydric aliphatic alcohol, dimethyl sulfoxide, acetone, protein-containing aqueous liquid, sugar-containing aqueous liquids, serum, plasma, liquid used in cell and tissue cultures and a mixture thereof.

Another object of the invention is to provide a method of producing an aqueous antimicrobial composition as defined in claims comprising coniferous resin acids, comprising the steps of:
- providing coniferous resin acids,
- dissolving the resin acids in a dispersing agent to provide a mixture,
- diluting the mixture with an aqueous medium to provide an aqueous antimicrobial composition,
- filtering the aqueous composition to provide the aqueous antimicrobial composition.

The antimicrobial composition of the invention can be used as an antimicrobial agent as such or as an additive in formulations used in human medicine and veterinary medicine. The formulations can contain the composition of the invention in therapeutically effective amounts or in amounts suitable for preservative purposes. The antimicrobial composition of the invention can also be used in industrial materials and products as well as in foodstuffs.

A further object of the invention is to provide an aqueous antimicrobial composition of the invention for use as an antimicrobial agent as such or as an additive in pharmaceutical formulations and products applied in human and/or veterinary medicines.

A still further object of the invention is to provide a pharmaceutical formulation comprising an aqueous antimicrobial composition of the invention and pharmaceutically acceptable diluent, carrier and/or excipient.

A still further object of the invention is to provide an aqueous antimicrobial composition of the invention for use in treating inflammation and/or infection in a mammal.

Said method can comprise administering a therapeutically effective amount of an aqueous antimicrobial composition of the invention.

A still further object of the invention is to provide a use of an aqueous antimicrobial composition of the invention as an antimicrobial agent in industrial materials and products, and in foodstuffs.

### Brief description of the drawings

Figures 1A and 1B show the antimicrobial effect of the aqueous antimicrobial compositions of the invention against *Staphylococcus aureus* in the agar diffusion plate test.
Figure 2 shows the antimicrobial effect of the aqueous antimicrobial compositions of the invention against *Helicobacter pylori* in Brucella agar plate.

### Detailed description of the invention

An object of the present invention is an aqueous antimicrobial composition as defined in claim 1 comprising coniferous resin acids, comprising hydroxylated derivatives of dehydroabietic acid, a dispersing agent and an aqueous medium. The composition is stable, evenly dispersible to and dilutable with an aqueous medium.

The content of the coniferous resin of the aqueous antimicrobial composition of the invention is in the range of 1 to 100 ppm. In an embodiment, the content is 1 to 50 ppm. In another embodiment, the content is 1 to 10 ppm.

The coniferous resin acids are selected from a group consisting of dehydroabietic acid, 7β-hydroxydehydroabietic acid, 7α-hydroxydehydroabietic acid, 15-hydroxydehydroabietic acid, 7β,15-dihydroxydehydroabietic acid, 7α,15-dihydroxydehydroabietic acid, 18-hydroxydehydroabietic acid, further hydroxylated derivates of dehydroabietic acid and a mixture thereof.

The coniferous resin acids are provided as or derived from "callus" resin of coniferous trees. In another embodiment, the coniferous resin acids are provided as or derived from a resin acid fraction of tall oil obtained from kraft pulping of coniferous trees. The coniferous resin acids can be provided as a solution or in powdery or granulated form.

The dispersing agent is selected from a group consisting of polyhydric alicyclic alcohol, polyhydric aliphatic alcohol, dimethyl sulfoxide, acetone, protein-containing aqueous liquid, sugar-containing aqueous liquid, serum, plasma, liquids used in cell and tissue cultures, such as agar, serum, artificial culture media, and a mixture thereof. In the context of the present invention, the term "polyhydric alcohol" means an alcohol having two or more hydroxyl groups in the molecule. In an embodiment, the polyhydric aliphatic alcohol is selected from a group consisting of glycerol, monopropylene glycol, xylitol, mannitol and sorbitol. In an embodiment of the invention, the dispersing agent is glycerol.

The aqueous medium can be any suitable aqueous solution. In an embodiment, the aqueous medium is a physiological saline solution. The physiological saline solution is preferred, when the antimicrobial composition is used in applications in fields of human and veterinary medicines. In another embodiment, the aqueous medium is water.

The antimicrobial effect of the composition of the invention can be adjusted by diluting the composition with the aqueous medium.

The composition of the invention is free of highly volatile organic compounds, such as ethanol.

In an aspect, the invention provides a method for producing an aqueous antimicrobial composition of the invention, comprising the steps of:
- providing coniferous resin acids,
- dissolving the resin acids in a dispersing agent to provide a mixture,
- diluting the mixture with an aqueous medium to provide an aqueous antimicrobial composition,
- filtering the aqueous composition to provide the aqueous antimicrobial composition.

The dispersing agent can contain an amount of an aqueous medium as long as the dispersing ability of the agent is still maintained. In an embodiment, the dispersing agent contains up to about 50% of an aqueous medium. In an embodiment, the dispersing agent contains up to about 30% of an aqueous medium.

If desired, the resin acids can be extracted in volatile carrier substance, such as ethanol, prior to blending with the dispersing agent. The volatile substance is evaporated from the composition before further use of the composition. Heating can be used to enhance the evaporation.

If desired, the dissolution is performed under stirring and/or heating to enhance the dissolution of the resin acids.

During dilution of the mixture of the resin acids and the dispersing agent with the aqueous medium, a deposit of insoluble resin is formed. The deposit is removed, by filtration. Finally, an evenly distributed and stable colloidal dispersion is obtained. This dispersion can be further diluted with aqueous solution without formation of any precipitations or deposits.

A wide range of various bacteria, such as *Helicobacter pylori,* can cause stomach infections (chronic gastritis) that can be treated with the antimicrobial compositions of the invention. Chronic gastritis in stomach mucosa is the fundamental reason for the most important gastric disorders, such as stomach cancer or peptic ulcer, atrophic gastritis and hypochlorhydric stomach. It is widely known that the treatment against *Helicobacter pylori* is problematic which often requires several re-treatments attempts and many antibiotics. *Helicobacter pylori* infections are typically treated also with colloidal bismuth compositions that topically (intragastrically) kill bacteria that grow and multiplicate on the stomach mucosa surface.

The aqueous antimicrobial composition of the invention can be used as such as an aqueous solution or it can be formulated to appropriate pharmaceutical water-dilutable formulations suitable for the treatment of mammalian inflammation and/or infection. The formulations can contain further ingredients typically used in pharmaceutical formulations, such as excipients, fillers, flavourings, etc.

The pharmaceutical product can be for example a bandage, dressing, plaster, tissue towel, etc.

The composition of the invention is suitable for use in medical field as follows:
- as an antimicrobial therapeutic agent (tablets, capsules, infusion and injections liquids, salves, drops, gels, suppositorium, etc.)
- as an intravenous or intra-arterial infusion for the treatment of septicaemia
- as an antimicrobial fluid to be injected into tissues, joint, cavities, tissues or abscesses
- as an antimicrobial solution for washing of tissue cavities (oral cavity, maxillary sinus, ear canal, abdominal cavity, pleural cavity, urinary bladder and urinary tract or vagina, fistulae, fissures, or ducts, etc.)
- as an antimicrobial composition in treatment of skin ulcers, sores, burns, or infections
- as an antimicrobial additive in bandages, dressings, tissue towels or products used in treatment of skin ulcers, sores, burns or infections
- as an antimicrobial composition for fungal nail or skin infections
- as eye drop, eye salve or eye washing solution
- as mouthwash
- as a vagitorium, salve, cream or solution in treatment of gynecological infections or diseases (bacterial and fungal infections)
- as an additive in water-dilutable salves, creams, solutions, lotions, gels, sprays, soaps or medicaments used in treatment, disinfection or washing of skin, skin wounds, skin lesions or infections
- as an antimicrobial additive in cosmetic or hygiene products, tissue papers, soaps or shampoos
- as an antimicrobial additive for preservation of medicines
- as an antimicrobial agent in the treatment of outer surfaces of a mammal, such as skin, hair, feet hoofs and udders
- as an antimicrobial additive in the washing and protection of outer surfaces of a mammal, such as skin, hair, feet hoofs and udders.

A further object of the invention is to provide an aqueous antimicrobial composition of the invention for use as an antimicrobial agent as such or as an additive in pharmaceutical formulations and products applied in human and/or veterinary medicines.

A still further object of the invention is to provide a pharmaceutical formulation comprising an aqueous antimicrobial composition of the invention and pharmaceutically acceptable diluent, carrier and/or excipient.

A still further object of the invention is to provide a use of an aqueous antimicrobial composition of the invention in the manufacture of a medicament for treating inflammation and/or infection in a mammal.

A still further object of the invention is to provide an aqueous antimicrobial composition of the invention for use in treating inflammation and/or infection in a mammal.

Said method can comprise administering a therapeutically effective amount of an aqueous antimicrobial composition of the invention.

The composition of the invention can be absorbed to a solid carrier, such as gelatin, cellulose, starch. etc. If desired, the amount of the resin acids in the carrier can be increased to amounts higher than 100 ppm by evaporation the aqueous component of the composition, e.g., by heating or warming.

The composition of the invention can be diluted to a desired concentration with a pharmaceutical carrier, such as salve base or aqueous solution, to provide a pharmaceutical formulation. In an embodiment, the composition of the invention is combined with an aqueous ointment/salve base to provide an eye ointment or salve. The content of the coniferous resin in the eye salve or drops is typically in the range of 1 to 30 ppm.

The aqueous antimicrobial composition or formulations of the invention containing said composition can contain one or more pharmaceutically active agent, such as hydrocortisone, cortisone, glucosamine and dexpanthenol.

The composition or the formulation of the invention can be administered orally, intravenously, intra-arterially, subcutaneously, intramuscularly, topically, as an injection or as an infusion.

A further object of the invention is to provide an aqueous antimicrobial composition of the invention for use as an antimicrobial agent in non-medical field, such as in industrial materials and products as well as in foodstuffs. In non-medical purposes, the composition of the invention can be used as follows:
- as an antimicrobial additive in paints, lacquers, varnish, wax, or various other cover materials
- as an antimicrobial composition for protection of paper, paperboard, cardboard, leather, textile, cloth, fur, glass, metal, rubber, wood or plastic,
- as an antimicrobial composition is medical instruments such as catheter, prosthesis, implants, etc.
- as an antimicrobial additive in cleaning compositions or devices, disinfectants and toiletry, such as shampoo, soap, deodorant, mouthwash and toothpaste
- as an antimicrobial additive in cosmetics,
- as an antimicrobial additive for sterilization of solutions, such as drinking water,
- as an antimicrobial composition in cesspools, sewers, swimming pools
- as an antimicrobial additive in foodstuffs.

The composition of the invention can be applied to the product by coating, spraying, impregnating, brushing, spreading, wetting, etc. Food products can be protected, for example, by spreading the composition of the invention on the surface of foodstuff or by including the composition into a food product. If desired, the composition can be washed out from the food product before use.

The following examples are given for further illustration of the invention.

### Examples

### Example 1

10 g of purified solid coniferous resin in powdery form was dissolved in 100 ml of pure glycerol for 3 weeks. The resultant glycerol solution was diluted with saline to provide a 50% aqueous colloidal dispersion. The dispersion was then filtered. A clear dispersion was obtained as a stock solution that was further diluted with an aqueous medium without any formation of precipitations.

The amounts of various resin acids of the stock solution were analyzed by the GC/MS method. The amounts of resin acids are the mean values of three separate assays. In calculation of the quantities of resin acids, calibration was done against heptadecanoic acid and isopimaric acid standards.

The resin acids and contents thereof are disclosed in Table 1.

**Table 1**

| Dissolved resin acid | Content (mg/L) (ppm) |
|---|---|
| dehydroabietic acid | 0.70 |
| 7β-hydroxyabietic acid | 1.49 |
| 7α-hydroxydeabietic acid | 3.00 |
| 15-hydroxydehydroabietic acid | 12.08 |
| 7β,15-hydroxydehydroabietic acid | 5.43 |
| 7α,15-hydroxydehydroabietic acid | 15.30 |

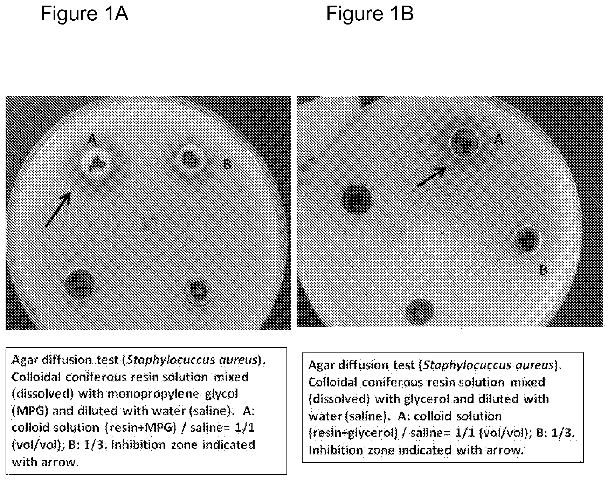

The stock solution was further diluted with a saline solution for the microbiological tests described below. Figure 1B illustrates the antimicrobial characteristic of the composition where the stock solution is diluted in the ratio of 1:1 by volume and 1:3 by volume with a physiological saline solution. In Table 2, the stock solution is diluted with a physiological saline solution in the range of 1:1-1:100 by volume.

For example, the stock solution can be diluted to a 25% solution with a physiological saline solution and can be used, for example, as an antimicrobial injection agent for inflamed joint, such as knee joint, or as an antimicrobial injection or washing agent in body cavities or abscesses. The solution can contain further appropriate pharmaceutically active agent(s), such as cortisone or glucosamine.

### Example 2

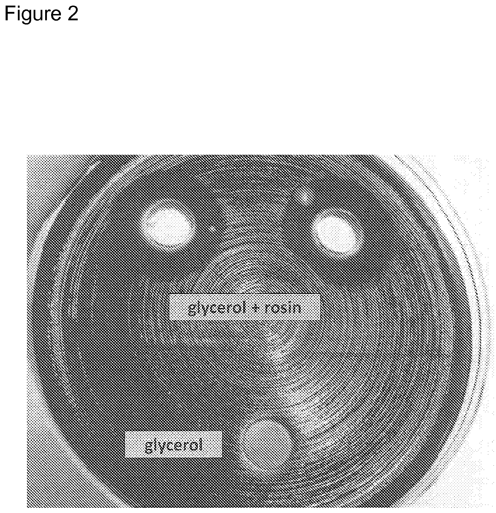

A stock solution was prepared as described in Example 1 except that rosin of industrial origin, that is for 90s from Forchem Oy, having high content of abietic type rosin acids, was used instead of spruce callus resin. The antimicrobial effect of the stock solution is shown in Table 2 and Figure 2.

The stock solution was further diluted with tap water in various ratios for the microbiological test described below. The results given in Table 3 are achieved with a solution where the stock solution is diluted with tap water in the ratio of 1:1 by volume. Table 4 shows the antimicrobial effect of the solutions with various dilutions.

### Example 3

A stock solution was prepared as described in Example 1 except that pure monopropylene glycol (MPG) was used as dispersing agent. The stock solution was diluted with a physiological saline solution in the ratio of 1:1 by volume and 1:3 by volume for the microbiological tests described below (Figure 1A).

The antimicrobial compositions can be blended with formulations suitable for use as an antimicrobial agent in the treatment of skin diseases, or as an antimicrobial additive in cosmetic products, soaps, shampoos or washing agent. The dispersion can also contain, for example, hydrocortisone, cortisone or dexpanthenol.

### Example 4

A stock solution was prepared as described in Example 1 except that xylitol was used as dispersing agent. The solution can be diluted with saline to provide an antimicrobial composition having 1 to 50 ppm of the resin acids. The composition can be used as mouthwash and as a solution for prevention and treatment of dental caries. The dispersion can be supplemented with therapeutically active agents and excipients, such as flavourings. The dispersion is applicable as an oral liquid formulation or as solid tablet where the composition is absorbed to a solid carrier (e.g. gelatin, agar) in the treatment of *H.pylori* infection.

### Example 5

Purified coniferous callus resin in powdery form was dissolved in an aqueous glycerol solution (70%) for several weeks to provide a dispersion. The dispersion was subsequently diluted with saline in the ratio of 1:2 by volume. The resultant solution is 25% with respect to glycerol. A precipitate of insoluble resin was formed and filtered off. A yellowish, clear, viscous solution was obtained. Its resin acid content is as described in Table 1 and the antimicrobial property as presented in Table 2.

### Example 6

Coniferous callus resin was dissolved in ethanol to provide a 65% solution. 40 ml of this ethanol solution was mixed for 1 day with 200 ml of pure glycerol to provide a mixture. The mixture was diluted with 240 ml of saline (1:1 by volume). A precipitation of insoluble resin acids was formed and removed by filtration. A colloidal dispersion was obtained as a filtrate. The filtrate was allowed to stand overnight at a room temperature to evaporate ethanol from the filtrate. The composition can be further diluted with an aqueous medium without precipitations.

### Example 7

Purified coniferous callus resin is dissolved in pure water for 2 hours. Microbiological challenge test in accordance with EN 13697 is performed. The solution showed no antimicrobial effect during 28 days against *S.aureus, E.coli, Ps.aeruginosa, Ps.putida, Kl.pneumoniae, C.ablicans* and *Aspergillus niger.* The example demonstrates that the using of a dispersion agent (e.g., glycerol) enhances the dissolvation of specific resin acids to water, resulting in as an antimicrobial dispersion composing of dispersion agent, water and specific water soluble resin acids in ppm quantity.

### Microbiological tests

The antimicrobial effect of the compositions of the invention is illustrated in Figures 1-2 and Tables 2-4. In Figures 1-2, the antimicrobial effect was tested be means of a diffusion test. In this test, the antimicrobial composition of the invention as a test solution is set into small, e.g., 2 mm, wells drilled into the medium on the plate. When the test solution diffuses from wells into the culture medium on the plate, it prevents the growth of the bacteria if the solution is antimicrobial. The antimicrobial effect is seen as a visible inhibition zone ("halo") of growth of bacteria around the well. The larger the inhibition zone is, the higher is the antimicrobial activity of the test solution.

In Figures 1A and 1B, the compositions of the invention prepared in Example 1 and 3, respectively, were tested against *Staphylococcus aureus* in the agar diffusion plate test, the microorganism being commonly used as a standard target in tests on antimicrobial influences. Figures 1A and 1B show that the compositions of the invention are antibacterial against the *Staphylococcus aureus.*

In Figure 2, the composition of the invention prepared in Example 1 was tested against *Helicobacter pylori* in Brucella agar plate. Figure 2 shows that the composition of the invention is clearly antimicrobial against the microorganism.

The antimicrobial effect of the compositions of the invention was also tested with a challenge test. The test was carried out in accordance with the European Pharmacopoeia microbiological challenge test (EN 13697). In the test, a specific number of test bacteria are inoculated into a specific volume of a solution tested for the antimicrobial property. After inoculation, samples are taken from the solution at specific time intervals and cultured in bacterium-specific media. By this setting, the antimicrobial activity of the solution can be calculated by indices of how much (how effectively) the test solution killed the inoculated bacteria in specific time unit. A reduction of the number of bacteria in colony forming unit more than 1 x log10 (4 logs) in 24 hours is considered to indicate a good antimicrobial activity.

In Table 2, the composition of the invention prepared in Examples 1 and 2 were tested against *Staphylococcus aureus* (MRSA strain). Test samples were analyzed after 24 hours incubation. The growth reduction of 1 indicates that the antimicrobial effect was poor. The growth reduction of > 4 logs indicates a reduction of number of microorganisms by more than 1 x 10log⁴ and means a good antimicrobial effect. The test showed that aqueous glycerol (70%) was not antimicrobial. The composition of the invention prepared in Example 1 was antimicrobial in saline dilutions up to 1:9. The composition of the invention prepared in Example 2 was also antimicrobial.

**Table 2.**

| Composition | Dilution | MRSA growth reduction |
|---|---|---|
| Aqueous glycerol (70%) | | 1 |
| Glycerol + resin + saline (Example 1) | 50% stock solution | > 4 log |
| | 1:1 | > 4 log |
| | 1:2 | > 4 log |
| | 1:3 | > 4 log |
| | 1:4 | > 4 log |
| | 1:5 | > 4 log |
| | 1:9 | > 4 log |
| | 1:15 | 1 |
| | 1:100 | 1 |
| Glycerol + rosin + saline (Example 2) | 50% stock solution | > 4 log |

The antimicrobial effect of the compositions of the invention was also tested against *Staphylococcus aureus* in accordance with the ISO standard 20645/20743 challenge test in accordance with EN 13697 on a steel plate. The test was performed after one week washing the steel plate with the composition. Test samples were analyzed after 24 hours incubation. The antimicrobial effect of the composition prepared in Example 2 is shown in Table 3 and 4.

**Table 3. Reduction in growth of microorganisms**

| | MRSA* | Enteroc. | PS. ae-rug. | E.coli | A.niger | C.albicans |
|---|---|---|---|---|---|---|
| Plate (ref.) | log 1 | log 1 | log 1 | log 1 | log 1 | log 1 |
| Plate washed with 70% ethanol | log 1 | log 1 | log 1 | log 1 | log 1 | log 1 |
| Plate washed with composition of Example 2 | > log 3 | > log 3 | > log 3 | > log 3 | > log 3 | > log 3 |

| | | | | | | |
|---|---|---|---|---|---|---|
| *Staphylococcus aureus | | | | | | |

The results show that the 70% ethanol exhibits no antimicrobial effect whereas the composition of Example 2 exhibits good antimicrobial effect.

**Table 4. Reduction in growth of staphylococcus aureus**

| Composition | Dilution | Growth reduction |
|---|---|---|
| 100% glycerol | | 1 |
| | 1:1 | 1 |
| | 1:3 | 1 |
| | 1:9 | 1 |
| | 1:16 | 1 |
| | 1:33 | 1 |
| Example 2 | 50% stock solution | log 4 |
| | 1:1 | > log 4 |
| | 1:3 | > log 4 |
| | 1:9 | > log 4 |
| | 1:16 | < log 4 |
| | 1:33 | < log 4 |

The results show that the 100% glycerol exhibits no antimicrobial effect whereas the composition of Example 2 exhibits good antimicrobial effect.

## Claims

1. An aqueous antimicrobial composition comprising coniferous resin acids, a dispersing agent and an aqueous medium, wherein an amount of the coniferous resin acids of the composition is in the range of 1 to 100 ppm, the coniferous resin acids comprise 7β-hydroxydehydroabietic acid, 7α-hydroxydehydroabietic acid, 15-hydroxydehydroabietic acid, 7β,15-dihydroxydehydroabietic acid, 7α,15-dihydroxydehydroabietic acid, 18-hydroxydehydroabietic acid, further hydroxylated derivates of dehydroabietic acid or a mixture thereof, and provided as or derived from resin of coniferous trees or are provided as or derived from a resin acid fraction of tall oil obtained from kraft pulping of coniferous trees, and the dispersing agent is selected from a group consisting of polyhydric alicyclic alcohol, polyhydric aliphatic alcohol, dimethyl sulfoxide, acetone, protein-containing aqueous liquid, sugar-containing aqueous liquids, serum, plasma, liquid used in cell and tissue cultures and a mixture thereof.

2. The composition of claim 1, wherein the amount of the coniferous resin acids of the composition is 1 to 50 ppm, more preferably 1 to 10 ppm.

3. The composition of claim 1 or 2, wherein the coniferous resin acids further comprise dehydroabietic acid.

4. The composition of any one of the preceding claims, wherein the polyhydric aliphatic alcohol is selected from a group consisting of glycerol, monopropylene glycol, xylitol, mannitol and sorbitol.

5. The composition of claim 4, wherein the dispersing agent is glycerol.

6. The composition of any one of the preceding claims, wherein the aqueous medium is water or a physiological saline solution.

7. A method for producing an aqueous antimicrobial composition of any one of claims 1 to 6, comprising the steps of:
- providing coniferous resin acids,
- dissolving the resin acids in a dispersing agent to form a mixture,
- diluting the mixture with an aqueous medium to provide an aqueous composition,
- filtering the aqueous composition to provide the aqueous antimicrobial composition.

8. The method of claim 7, wherein the dissolution is performed under stirring and/or heating.

9. A pharmaceutical formulation comprising an aqueous antimicrobial composition of any one of claims 1 to 6 or prepared by claim 7 or 8 and pharmaceutically acceptable diluent, carrier and/or excipient.

10. The pharmaceutical formulation of claim 9, wherein the formulation is a tablet, capsule, solution, infusion or injection liquid, clysma (enema), washing agent, drop, spray, salve, ointment, lotion, lacquer, enema, vagitorioum or suppositorium.

11. The pharmaceutical formulation of claim 9 or 10, wherein the formulation is administered orally, intravenously, intra-arterially, subcutaneously, intramuscularly, topically, as an injection or as an infusion.

12. A non-therapeutic use of an aqueous antimicrobial composition of any one of claims 1 to 6 or prepared by claim 7 or 8 as an antimicrobial agent as such or as an additive in pharmaceutical formulations and products applied in human and/or veterinary medicines; in industrial materials and products; in foodstuffs; for sterilization of water, water reservoirs, swimming pools, sewers; as a preservative in foodstuffs; or as a biocide.

13. An aqueous antimicrobial composition of any one of claims 1 to 6 or prepared by claim 7 or 8 for use in treating inflammation and/or infection in a mammal.

14. The composition of claim 13, wherein the inflammation and/or infection is caused by *Helicobacter pyroli.*

## Patentansprüche

1. Wässrige antimikrobielle Zusammensetzung, umfassend Nadelharzsäuren, ein Dispergiermittel und ein wässriges Medium, wobei eine Menge der Nadelharzsäuren der Zusammensetzung im Bereich von 1 bis 100 ppm liegt, die Nadelharzsäuren 7β-Hydroxydehydroabietinsäure, 7α-Hydroxydehydroabietinsäure, 15-Hydroxydehydroabietinsäure, 7β,15-Dihydroxydehydroabietinsäure, 7α,15-Dihydroxydehydroabietinsäure, 18-Hydroxydehydroabietinsäure, weitere hydroxylierte Derivate von Dehydroabietinsäure oder ein Gemisch davon umfassen, welche als Nadelharzsäuren bereitgestellt werden oder davon abgeleitet sind oder als Harzsäurefraktion von Tallöl bereitgestellt werden oder daraus abgeleitet sind, die aus einem Kraftaufschluss von Nadelbäumen erhalten wird, und das Dispergiermittel ausgewählt ist aus der Gruppe bestehend aus mehrwertigem alicyclischem Alkohol, mehrwertigem aliphatischem Alkohol, Dimethylsulfoxid, Aceton, proteinhaltigen wässrigen flüssigen, zuckerhaltigen wässrigen Flüssigkeiten, Serum, Plasma, Flüssigkeit, die in Zell- und Gewebekulturen verwendet wird, und einer Mischung davon.

2. Zusammensetzung nach Anspruch 1, wobei die Menge der Nadelharzsäuren der Zusammensetzung 1 bis 50 ppm, mehr bevorzugt 1 bis 10 ppm, ist.

3. Zusammensetzung nach Anspruch 1 oder 2, wobei die Nadelharzsäuren ferner Dehydroabietinsäure umfassen.

4. Zusammensetzung nach einem der vorherigen Ansprüche, wobei der mehrwertige aliphatische Alkohol ausgewählt ist aus der Gruppe bestehend aus Glyzerin, Monopropylenglykol, Xylitol, Mannitol und Sorbitol.

5. Zusammensetzung nach Anspruch 4, wobei das Dispergiermittel Glyzerin ist.

6. Zusammensetzung nach einem der vorherigen Ansprüche, wobei das wässrige Medium Wasser oder eine physiologische Kochsalzlösung ist.

7. Verfahren zum Erzeugen einer wässrigen antimikrobiellen Zusammensetzung nach einem der Ansprüche 1 bis 6, umfassend die Schritte:
- Bereitstellen von Nadelharzsäuren,
- Auflösen der Nadelharzsäuren in einem Dispergiermittel, um eine Mischung zu bilden,
- Verdünnen der Mischung mit einem wässrigen Medium, um eine wässrige Zusammensetzung bereitzustellen,
- Filtrieren der wässrigen Zusammensetzung, um die wässrige antimikrobielle Zusammensetzung bereitzustellen.

8. Verfahren nach Anspruch 7, wobei die Auflösung unter Rühren und/oder Erwärmen durchgeführt wird.

9. Pharmazeutische Formulierung umfassend eine wässrige antimikrobielle Zusammensetzung nach einem der Ansprüche 1 bis 6 oder hergestellt nach Anspruch 7 oder 8 und ein pharmazeutisch verträgliches Verdünnungsmittel, einen pharmazeutisch verträglichen Träger und/oder Hilfsstoff.

10. Pharmazeutische Formulierung nach Anspruch 9, wobei die Formulierung eine Tablette, Kapsel, Lösung, Infusions- oder Injektionsflüssigkeit, Clysma (Einlauf), Waschmittel, Tropfen, Spray, Salbe, Salbe, Lotion, Lack, Einlauf, Vagitorium oder Suppositorium ist.

11. Pharmazeutische Formulierung nach Anspruch 9 oder 10, wobei die Formulierung oral, intravenös, intra-arteriell, subkutan, intramuskulär, topisch, als Injektion oder als Infusion verabreicht wird.

12. Nicht-therapeutische Verwendung einer wässrigen antimikrobiellen Zusammensetzung nach einem der Ansprüche 1 bis 6 oder hergestellt nach Anspruch 7 oder 8 als antimikrobielles Mittel als solches oder als Additiv in pharmazeutischen Formulierungen und Produkten, die in humanen und/oder veterinären Arzneimitteln, in industriellen Materialien und Produkten; in Lebensmitteln; zur Sterilisierung von Wasser, Wasserspeichern, Schwimmbecken, Abwasserkanälen; als Konservierungsmittel in Lebensmitteln; oder als Biozid.

13. Wässrige antimikrobiellen Zusammensetzung nach einem der Ansprüche 1 bis 6 oder hergestellt nach Anspruch 7 oder 8 zur Verwendung im Behandeln einer Entzündung und/oder Infektion in einem Säuger.

14. Zusammensetzung nach Anspruch 13, wobei die Entzündung und/oder Infektion durch *Helicobacter pyroli* verursacht wird.

## Revendications

1. Composition aqueuse antimicrobienne comprenant des acides résiniques de conifères, un agent dispersant et un milieux aqueux, dans laquelle une quantité d'acides résiniques de conifères de la composition est située dans la gamme de 1 à 100 ppm, les acides résiniques de conifères comprenant l'acide 7β-hydroxydéshydroabiétique, l'acide 7α-hydroxydéshydroabiétique, l'acide 15-hydroxydéshydroabiétique, l'acide 7β,15-dihydroxydéshydroabiétique, l'acide 7α,15-dihydroxydéshydroabiétique, l'acide 18-hydroxydéshydroabiétique, également des dérivés hydroxylés de l'acide déshydroabiétique ou leur mélange, et étant fournis comme ou dérivés de résine de conifères ou étant fournis ou dérivés d'une fraction d'acide résinique d'huile de tall obtenus à partir de pâtes kraft de conifères, et l'agent dispersant est choisi dans le groupe constitué par un polyol alicyclique, un polyol aliphatique, le diméthylsulfoxyde, l'acétone, un liquide aqueux contenant des protéines, les liquides aqueux contenant des sucres, le sérum, le plasma, un liquide utilisé dans les cultures cellulaires ou tissulaires et leurs mélanges.

2. Composition selon la revendication 1, dans laquelle la quantité d'acides résiniques de conifères de la composition est de 1 à 50 ppm, plus préférentiellement de 1 à 10 ppm.

3. Composition selon la revendication 1 ou 2, dans laquelle les acides résiniques de conifères comprennent en outre l'acide déshydroabiétique.

4. Composition selon l'une des revendications précédentes, dans laquelle le polyol aliphatique est choisi dans le groupe constitué par le glycérol, le monopropylène glycol, le xylitol, le mannitol et le sorbitol.

5. Composition selon la revendication 4, dans laquelle l'agent dispersant est le glycérol.

6. Composition selon l'une des revendications précédentes, dans laquelle le milieu aqueux est l'eau ou une solution saline physiologique.

7. Procédé de production d'une composition aqueuse antimicrobienne selon l'une quelconque des revendications 1 à 6, comprenant les étapes :
- de fourniture d'acides résiniques de conifères,
- de dissolution des acides résiniques dans un agent dispersant pour former un mélange,
- de dilution du mélange par un milieu aqueux pour fournir une composition aqueuse,
- de filtration de la composition aqueuse pour fournir la composition aqueuse antimicrobienne.

8. Procédé selon la revendication 7, dans lequel la dissolution est effectuée sous agitation et/ou chauffage.

9. Formulation pharmaceutique comprenant une composition aqueuse antimicrobienne selon l'une quelconque des revendications 1 à 6 ou préparée selon la revendication 7 ou 8 et un diluant, un vecteur et/ou un excipient acceptable(s) sur le plan pharmaceutique.

10. Formulation pharmaceutique selon la revendication 9, dans laquelle la formulation est un comprimé, une gélule, une solution, un liquide de perfusion ou d'injection, un clystère, un agent de lavage, une goutte, une pulvérisation, un baume, un onguent, une lotion, une laque, un énéma, un pessaire ou un suppositoire.

11. Formulation pharmaceutique selon la revendication 9 ou 10, dans laquelle la formulation est administrée de façon orale, intraveineuse, intra-artérielle, sous-cutanée, intramusculaire, topique, comme injection ou comme perfusion.

12. Utilisation non thérapeutique d'une composition aqueuse antimicrobienne selon l'une quelconque des revendications 1 à 6 ou préparée selon la revendication 7 ou 8 comme agent antimicrobien en tant que tel ou comme additif dans les formulations et produits pharmaceutiques appliqués aux êtres humains et/ou dans les médicaments à usage vétérinaire ; dans les matières ou produits industriels ; dans les produits alimentaires ; pour la stérilisation de l'eau, des réservoirs d'eau, des piscines, des eaux usées ; comme conservateur dans les produits alimentaires ; ou comme biocide.

13. Composition aqueuse antimicrobienne selon l'une quelconque des revendications 1 à 6 ou préparée selon la revendication 7 ou 8 destinée à être utilisée dans le traitement d'une inflammation et/ou d'une infection chez un mammifère.

14. Composition selon la revendication 13, dans laquelle l'inflammation et/ou l'infection est provoquée par la *Helicobacter pyroli.*
